Europäisches Patentamt

European Patent Office

Office européen des brevets

(19)

(11) **EP 0 877 913 B1**

(12) **EUROPÄISCHE PATENTSCHRIFT**

(45) Veröffentlichungstag und Bekanntmachung des Hinweises auf die Patenterteilung:
**09.10.2002 Patentblatt 2002/41**

(21) Anmeldenummer: **96910889.3**

(22) Anmeldetag: **06.05.1996**

(51) Int Cl.$^7$: **G01B 9/02**, A61B 3/10

(86) Internationale Anmeldenummer:
**PCT/CH96/00172**

(87) Internationale Veröffentlichungsnummer:
**WO 96/035100 (07.11.1996 Gazette 1996/49)**

(54) **VORRICHTUNG ZUR MESSUNG DER DICKE TRANSPARENTER GEGENSTÄNDE**

DEVICE FOR MEASURING THE THICKNESS OF TRANSPARENT OBJECTS

DISPOSITIF POUR LA MESURE D'EPAISSEUR D'OBJETS TRANSPARENTS

(84) Benannte Vertragsstaaten:
**AT CH DE ES FR GB IE IT LI NL**

(30) Priorität: **04.05.1995 CH 128495**

(43) Veröffentlichungstag der Anmeldung:
**18.11.1998 Patentblatt 1998/47**

(73) Patentinhaber: **Haag-Streit AG**
**3098 Köniz (CH)**

(72) Erfinder:
• **CHAVANNE, Philippe**
**CH-1123 Aclens (CH)**
• **SALATHE, René, Paul**
**CH-1122 Romanel-sur-Morges (CH)**

(74) Vertreter:
**Roshardt, Werner Alfred, Dipl.-Phys. et al**
**Keller & Partner**
**Patentanwälte AG**
**Schmiedenplatz 5**
**Postfach**
**3000 Bern 7 (CH)**

(56) Entgegenhaltungen:
**EP-A- 0 636 858        GB-A- 2 069 169**

• **APPLIED OPTICS, Bd. 30, Nr. 21, 1991 - 20.Juli 1991, NEW YORK, NY, USA, Seiten 2975-2979, XP000216446 B.L. DANIELSON, C.Y. BOISROBERT: "Absolute Optical Ranging using Low Coherence Interferometry"**

**Beschreibung**

Die Erfindung betrifft eine Vorrichtung gemäß dem Oberbegriff des Patentanspruchs 1 und eine Verwendung der Vorrichtung gemäß dem Patentanspruch 10.

Es ist bekannt, zur Messung der Dicke transparenter Gegenstände ein Interferometer, insbesondere ein Michelson-Interferometer, zu verwenden. Dieses Interferometer weist einen Strahlteiler, einen sog. Koppler auf, der einen von einer Strahlungsquelle ausgehenden Strahl in zwei Teilstrahlen, einen Meß- und einen Referenzstrahl, aufspaltet, welche nach einer vorgegebenen Weglänge in sich zurückgesandt, im Strahlteiler überlagert und dann teilweise in die Strahlungsquelle zurückgesandt bzw. in einen Beobachtungsdetektor eingestrahlt werden. Wird nun eine Strahlungsquelle verwendet, deren Strahlung eine sehr kurze Kohärenzlänge aufweist, so wird Interferenz zwischen Referenz- und Meßstrahl nur dann erhalten, wenn deren beide Wege exakt gleich lang sind. Diese Interferenz läßt sich mit dem Beobachtungsdetektor feststellen.

Zur Bestimmung von Abstandswerten eines Objektes, dessen Grenzfläche lediglich nur einen geringen Bruchteil der Strahlung zu reflektieren braucht, wird vom Strahlteiler ausgehend nun die Länge des Referenzweges mit einer möglichst konstanten Veränderungsgeschwindigkeit variiert, bis Interferenz der Strahlung von Referenz- und Meßweg eintritt. Je nach Veränderungsgeschwindigkeit erleidet die Strahlungsfrequenz f der im Referenzweg laufenden Strahlungswelle eine Dopplerverschiebung gemäß der Relation

$$\frac{\blacktriangle f}{f_0} = \frac{2v}{c},$$

wobei $f_0$ die ursprüngliche Strahlungsfrequenz, c die Lichtgeschwindigkeit und v die Geschwindigkeit der Veränderung der optischen Weglänge $\blacktriangle s$ - der optischen Weglängenänderung pro Zeiteinheit - ist.

Ist nun mit dem Beobachtungsdetektor eine Interferenzerscheinung bei gleichen Wegen von Referenz- und Meßweg feststellbar, so ändert sich das Interferenzmuster gemäß der obigen Dopplerverschiebung mit der Frequenz $\blacktriangle f$. Bei einer streng linearen Veränderung der Weglängendifferenz ist $\blacktriangle f$ zeitlich konstant. Treten nichtlineare Weglängenänderungen auf, so variiert $\blacktriangle f$ innerhalb eines Frequenzbands.

Michelson-Interferometer, bei denen Weglängenveränderungen im Referenzweg erzeugt wurden, sind u. a. aus der EP-A 0 529 603, der EP-A 0 443 477 und der EP-A 0 449 335 bekannt. In der EP-A 0 529 603 war in jedem Arm des Interferometers ein Retroreflektor angeordnet. Jeder der beiden Retroreflektoren war über ein einen Zahnriemen aufweisendes Getriebe derart verstellbar, daß die jeweilige Vorderseite des Retroreflektors zur Oberfläche eines feststehenden Reflektors auch bei einer Abstandsänderung zu einem feststehenden Reflektor parallel blieb. Die Abstandsveränderung erfolgte nun derart, daß die optische Weglänge sich in den einen Interferenzarm vergrößerte, während sie sich in dem anderen gerade verkürzte. Da die beiden Antriebe synchronisiert sein mußten, erfolgte der Antrieb über elektrische Schrittmotoren. Diese Anordnung war aufwendig und wurde zudem durch die elektrischen Impulse der Schrittmotoren leicht in störende mechanische Schwingungen versetzt.

In der EP-A 0 443 477 wurden pro Interferenzarm zwei zueinander gegensinnig rotierende Retroreflektoren, je ein die Strahlung umlenkender weiterer Retroreflektor sowie je ein feststehender Planspiegel verwendet. Auch hier war eine Synchronisation zwischen der Rotation der Retroreflektoren notwendig, welche auch hier mit mechanischen Schwingungen verursachenden Schrittmotoren vorgenommen wurde.

Zur Veränderung der Weglängendifferenz in den beiden Interferenzarmen wurde in der EP-A 0 449 335 je eine dicke transparente, planparallele Platte verwendet. Die Platte wurde nun bei dem Hin- sowie bei dem Rückweg nach Reflexion am feststehenden Reflektor von der Strahlung einmal durchlaufen, wobei sich der optische Weg je nach Neigung der Platte veränderte.

Die bekannten Elemente zur Veränderung der Weglängendifferenz zwischen Meß- und Referenzstrahl weisen entweder eine komplizierte mechanische und damit erschütterungsanfällige Anordnung und/oder eine erhebliche nichtlineare Wegdifferenzänderung auf.

Aufgabe der Erfindung ist es, eine Vorrichtung zu schaffen, bei der mit einer einfachen und störungssicheren Anordnung eine gute Meßgenauigkeit bei der Messung der Dicke transparenter Gegenstände erreichbar ist.

Die Aufgabe wird dadurch gelöst, daß im Gegensatz zu den bekannten Vorrichtungen im Referenzstrahlengang nicht mehr ein oder mehrere Retroreflektoren oder eine nur einfach durchstrahlte planparallele Platte verwendet wird, sondern ein mehreckiges, wenigstens vier Ecken aufweisendes, transparentes, rotierendes Element verwendet wird. Die Abmessungen der Seitenflächen des Elements, der Einstrahlungsort auf diesen sowie der Brechungsindex des Elementmaterials werden nun derart gewählt, daß in einer bevorzugten Ausführungsform die mit dem rotierenden Element erreichbare, sich zeitlich ändernde Weglängendifferenz über dem Drehwinkel angenähert linear verläuft.

Die erfindungsgemäße Vorrichtung eignet sich insbesondere zur Dickenbestimmung transparenter Medien, deren Flächen einer mechanischen Messung nicht oder nur schwer zugänglich sind. Sie gestattet auch Dickenmessungen, bei denen die Grenzflächen nur geringe Reflektivität aufweisen. Hervorzuheben ist noch die große Abtastgeschwindigkeit der erfindungsgemäßen Vorrichtung, welche auch Messungen an bewegten Objekten zuläßt sowie auch zur Überwachung, insbesondere zeitlich rascher materialabtragender oder -auftragender Prozesse am auszumessenden Gegenstand geeignet ist.

Ergänzend wird darauf hingewiesen, daß das die Weglängenänderung verursachende rotierende Element in der Meßeinheit sowie in der Referenzeinheit angeordnet werden kann, da es lediglich auf eine Weglängendifferenz in den beiden Armen (Meßarm/Referenzarm) ankommt. Auch können zwei, bevorzugt gegenläufig rotierende Elemente verwendet werden, wobei dann in jedem Arm eines angeordnet wird. Hierdurch ist eine Vergrößerung des Variationsbereichs und der Abtastgeschwindigkeit erreichbar. Die exakte Winkelstellung des Elements, welche die additive Weglänge angibt ist z. B. mittels bekannter Codierscheiben ermittelbar.

Durch die Faltung des Strahls im Element infolge von Mehrfachreflexionen wird mit einem verhältnismäßig kleinen Raumvolumen eine große Änderungsmöglichkeit der Wegdifferenz erreicht.

Auch wird im Gegensatz zu den bekannten Vorrichtungen nur ein einziges, einfach anzutreibendes, um eine Achse rotierendes Element verwendet. Als Antrieb kann ein erschütterungsfreier Gleichstrommotor verwendet werden. Ein zu überwachender und zu steuernder Synchronlauf mehrerer Elemente entfällt somit und damit auch die Erzeugung mechanischer Erschütterung, wie sie sich durch die verwendeten Schrittmotoren bei den bekannten Vorrichtungen ergeben.

Durch die Verwendung eines Strahlungsleiters, insbesondere im Meßarm, ist eine problemlose Ankopplung des auszumessenden Gegenstands (Objekts) gegeben. Wird auch der Referenzarm mit einem Strahlungsleiter versehen, so ist eine einfache und raumsparende Wegangleichung der beiden Strahlungswege möglich. Bei Bestimmung der Dicke transparenter Gegenstände wird jeweils eine Interferenz zwischen der Referenzstrahlung und der an den Oberflächen des Meßobjekts (Gegenstands) reflektierten Meßstrahlung erhalten. Der Übergang von einem Medium in ein anderes mit unterschiedlichem Brechungsindex erzeugt die reflektierte Meßstrahlung.

Aufgrund der unten beschriebenen Geometrie sowie einer optimierten Auswahl der Abmessungen, des Einstrahlungsorts sowie des Brechungsindexes des rotierenden Elements läßt sich eine ausgezeichnete Linearität der Weglängenänderung erreichen, was wiederum eine geringe Bandbreite der Dopplerfrequenz ergibt. Diese geringe Bandbreite der Dopplerfrequenz gestattet eine ausgezeichnete Filterung und ergibt somit ein großes Signal-Rausch-Verhältnis. Aufgrund dieses großen erreichbaren Signal-Rausch-Verhältnisses ist es möglich, Dicken von transparenten Gegenständen auszumessen, bei denen eine Brechungsindexänderung zwischen dem Material des auszumessenden Objekts und der Umgebung sehr gering ist. Die unten beschriebene Meßanordnung ist derart empfindlich, daß die Dicken von Objekten bestimmbar sind, welche z. B. an der Vorderfläche eine Strahlungsreflexion von 4% und an der Hinterfläche einen Reflexionsgrad von lediglich $10^{-8}$ aufweisen. Auch kann der Reflexionsgrad der Vorderfläche hoch, wie z. B. bei einem Metallspiegel sein; es ist eine Messung durch diesen Metallspiegel hindurch möglich. Auch bei einer kleinen Vorderflächenreflexion und einer großen Hinterflächenreflexion kann mit der erfindungsgemäßen Vorrichtung gearbeitet werden. Die Erfindung eignet sich insbesondere zum Ausmessen der Kornea des menschlichen Auges, welche eine Vorderflächenreflexion von 2,5 % und eine Hinterflächenreflexion von $2,2 \cdot 10^{-4}$ hat.

Im folgenden werden Beispiele der erfindungsgemäßen Vorrichtung anhand von Zeichnungen näher erläutert. Weitere Vorteile der Erfindung ergeben sich aus dem nachfolgenden Beschreibungstext. Es zeigen:

Fig. 1     ein Blockschaltbild einer Vorrichtung zur Messung der Dicke transparenter Gegenstände,

Fig. 2     eine vergrößerte Darstellung des Weglängenvariationselements mit Reflektor im Referenzarm der in **Figur 1** dargestellten Vorrichtung, wobei gerade eine Seite des Weglängenvariationselements parallel zur Reflektoroberfläche steht,

Fig. 3     eine zur Figur 2 analoge Darstellung des Elements mit Strahlengang, jedoch gegenüber der Darstellung in **Figur 2** um 20° und in

Fig. 4     um 40° verdreht, wobei die Beschichtungen **40**a bis **40d** in den **Figuren 2** bis **4** zur Kenntlichmachung stark hervorgehoben sind,

Fig. 5     in der oberen Abbildung die Weglängendifferenz ▲s [mm] über den Drehwinkel $\alpha$ des Elements mit einer Seitenlänge $l_k$ von 30 mm, einem Abstand **e** von 13 mm und einem Brechungsindex $n_e$ des Materials des Elements von 1,5, wobei die gestrichelten Kurvenwerte Werte angeben, welche mit den in den **Figuren 2** bis **4** gezeigten Elementen nicht erreichbar sind, da die Elementoberflächen ausgehend von jeweils einer Elementkante eine teilweise Reflexbeschichtung aufweisen; die untere Kurve zeigt die hierzu gehörende Weglängenänderung ▲s/t in Abhängigkeit des Drehwinkels,

Fig. 6    eine zu **Figur 5** analoge Darstellung, jedoch für einen Brechungsindex von $n_e = 2,5$,

Fig. 7    eine zu den **Figuren 3** bis **4** analoge Darstellung für ein Oktogon als Weglängenvariationselement und

Fig. 8    die zur **Figur 5** analoge Darstellung für das in Figur 7 dargestellte Weglängenvariationselement.

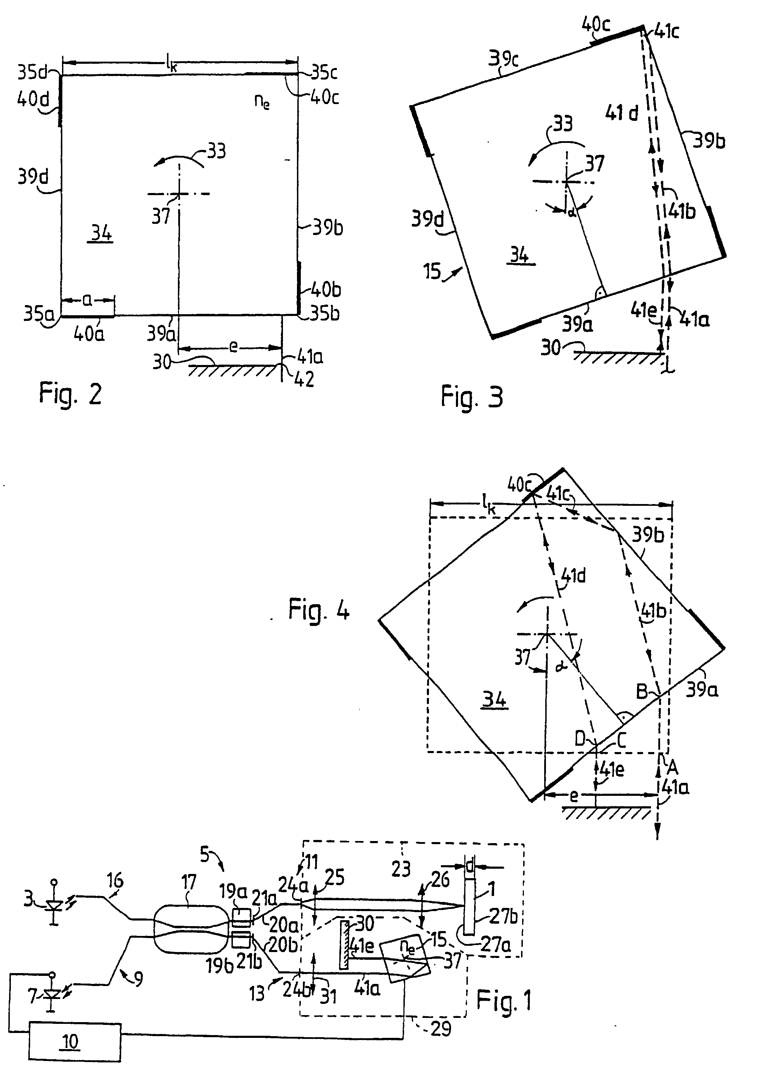

Die in **Figur 1** als Blockschaltbild dargestellte Vorrichtung zur Messung der Dicke **d** eines transparenten Gegenstands **1** hat als Strahlungsquelle **3** mit sehr kurzer Kohärenzlänge im Bereich von etwa 10 bis 15 μm eine Super-Lumineszenzdiode, ein Michelson-Interferometer **5** und im Beobachtungsarm **9** einen mit einer Auswerteeinheit **10** zusammenarbeitenden Strahlungsdetektor **7**. Im Meßarm **11** des Interferometers **5** ist der Gegenstand **1** und im Referenzarm **13** ein in Rotation versetzbares Weglängenvariationselement **15** angeordnet.

Die Meß-, Referenz-, Beleuchtungs- und Beobachtungsarme **11**, **13**, **16** bzw. 9 sind über einen 50/50%-Koppler **17** miteinander verbunden. Im Meß- und Referenzarm **11** und **13** ist vor dem Anschluß an den Koppler **17** je eine Polarisationskontrolleinheit **19a** bzw. **19b** angeordnet. An die Polarisationskontrolleinheit **19a** im Meßarm **11** ist ein Strahlungsleiter **20a** mit einer lösbaren Kupplung **21a** angeschlossen, der zu einer Meßeinheit **23** führt. Die Meßeinheit **23** ist mit dem anderen Ende des Strahlungsleiters **20a** ebenfalls über eine lösbare Kupplung **24a** verbunden. Die Meßeinheit **23** hat eine Linse **25** zur Kollimierung der durch den Strahlungsleiter **20a** geführten Strahlung sowie eine Fokussierlinse **26** zur Fokussierung der ausgesandten Strahlung und zum Sammeln der von den Gegenstandsoberflächen **27a** und **27b** reflektierten Strahlung. Die Fokussierlinse **26** wird bevorzugt derart angeordnet, daß hier Strahlung von der hinteren Oberfläche **27b** mit dem sehr kleinen Reflexionsgrad - siehe bevorzugter Verwendungszweck - gebündelt wird.

An die Polarisationskontrolleinheit **19b** des Referenzarms **13** ist ebenfalls ein Strahlungsleiter **20b** mit einer lösbaren Kupplung **21b** angeschlossen, der zur Referenzeinheit **29** mit dem Weglängenvariationselement **15** und einem nachgeschalteten Reflektor **30** führt. Das andere Ende des Strahlungsleiters **20b** ist ebenfalls mit der Referenzeinheit **29** über eine lösbare Kupplung **24b** verbunden. In der Referenzeinheit **29** wird die durch den Strahlungsleiter **20b** geführte Strahlung mit einer Linse **31** kollimiert in das Element **15** eingestrahlt.

Die Strahlungsführung im Referenz- und im Meßarm **13** und **11** wird bevorzugt derart gewählt, daß die Unterschiede in der Dispersion in beiden Armen **11** und **13** vernachlässigbar ist, damit keine Verbreiterung des Interferenzsignals auftritt.

In den **Figuren 2** bis **4** sind Anordnungen des Weglängenvariationselements **15** mit Reflektor **30** in gegenüber **Figur 1** vergrößerter Darstellung gezeigt. Das Element **15** ist durch einen nicht dargestellten Antrieb in Rotation, gemäß Pfeil **33**, versetzbar. Die Querschnittsfläche **34** des Elements **15**, in welcher der Referenzstrahl **41b**, **41c** und **41d** im Element **15** zu liegen kommt, weist vier Ecken **35a** bis **35d** auf und ist in dem hier ausgewählten Beschreibungsbeispiel quadratisch ausgebildet, d. h. das Element ist ein gerader Zylinder mit quadratischer Grundfläche. Die Rotationsachse **37** ist mit der Achse des Zylinders identisch. Jede Zylindermantelfläche **39a** bis **39d** des Elements **15a** ist mit einer teilweisen Beschichtung **40a** bis **40d** versehen, welche derart ausgewählt ist, daß sie die im Element **15** befindlichen Strahlen **41c** bzw. die rückreflektierten Strahlen **41d** der Strahlung optimal reflektiert. Am Reflexionsort des Strahls **41b** bzw. des rückreflektierten Strahls **41c** wird an der hier beispielsweise gezeichneten Wand **39b** keine Reflexionsbeschichtung benötigt, da hier Totalreflexion erfolgt. Die Beschichtungen **40a** bis **40d** beginnen jeweils an den Ecken (Kanten) **35a** bis **35d** und erstrecken sich über eine Distanz a in die betreffende Seitenfläche **39a** bis **39d** hinein. Ausgehend von jeder Kante **35a** bis **35d** ist jeweils nur eine der beiden anstoßenden Seiten beschichtet, und zwar jeweils nur immer diejenige, auf der der reflektierte mit dem einfallenden Strahl einen spitzen Winkel bildet, siehe hierzu inbesondere Figur 3.

Bei der in **Figur 2** dargestellten Augenblicksstellung des rotierenden Elements **15** ist dessen Seite **39a** parallel zur Oberfläche des Reflektors **30** stehend dargestellt. Der in das Element **15** eintretende Referenzstrahl **41a** ist derart geführt, daß er gerade an der in den **Figuren 2, 3** und **4** rechten Kante **42** des Reflektors **30** vorbeiführbar ist. Der Abstand e von der Reflektorkante **42** ist gerade so groß gewählt, daß er nur geringfügig kleiner als die halbe Würfelkante ist. Bei dem hier gewählten Zahlenbeispiel mit einer Flächenbreite $l_k$ von 30 mm, wird der Referenzstrahl **41a** in einem Abstand **e** von 13 mm von der zentrischen Rotationsachse **37** und in einem Abstand von etwa 3 mm von der Reflektorkante 42 entfernt eingestrahlt.

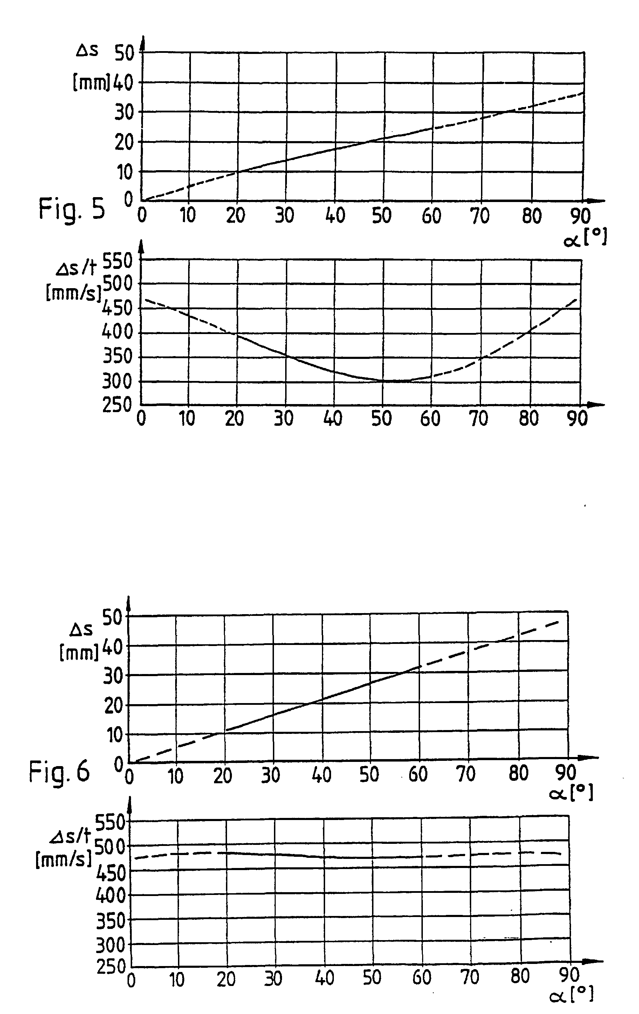

**Figur 3** zeigt das Element **15** gegenüber der Darstellung in **Figur 2** in einer um einen beispielsweisen Winkel α von 20° verdreht. Der Strahl **41a** dringt unter Brechung als Strahl **41b** in das transparente Medium des Elements **15** ein und wird an der Fläche **39b** total reflektiert. Der auftreffende und der reflektierte Strahl **41b** und **41c** bilden miteinander einen stumpfen Winkel. Der reflektierte Strahl **41c** trifft auf die Innenseite der Fläche **39c** und wird dort durch den beschichteten Bereich **40c** als Strahl **41d** in Richtung Fläche **39a** parallel zum Strahl **41b** reflektiert. Die beiden Strahlen **41c** und **41d** bilden einen spitzen Winkel miteinander. Der auf die Fläche **39a** auftreffende Strahl **41d** wird gebrochen, trifft als Strahl **41e** senkrecht auf den Reflektor **30** und wird dort unter Totalreflexion wieder in sich selbst zurückreflektiert, so daß der am Reflektor **30** reflektierte Strahl am Ort des Eintritts des Strahls **41a** mit derselben Richtung wie der Strahl **41a** diesen verläßt. Wie im Vergleich der **Figuren 3** und **4** zu ersehen ist, wandert der Reflexionspunkt des Strahls **41e** auf dem Reflektor **30** hin und her.

Die Weglängenveränderung ▲s infolge der Rotation des Elements **15** setzt sich nun aus dem doppelten Weg der sich ändernden Weglängen der Strahlen **41a** bis **41e** zusammen. Für die Strahlen **41b** bis **41d** ist zu beachten, daß sich deren Weglängen durch den Brechungsindex n des Mediums, in dem sie laufen, erhöht sind. Gemäß **Figur 4** ist eine Weglänge **s** abhängig vom Drehwinkel $\alpha$, vom Abstand des Strahleintritts **e**, vom Brechungsindex $\mathbf{n_e}$ und der Flächenbreite $\mathbf{l_k}$ der Flächen **39a** bis **39d**. Die unten berechnete Weglänge s beginnt und endet an der gestrichelten Geraden A-D in **Figur 4**, welche die in **Figur 2** gezeigte Lage des Elements **15** wiedergibt.

$$s = n_e \cdot (41b + 41c + 41d) + CD + AB$$

Da

$$41b + 41c = 41d$$

ist, folgt

$$s = 2 \cdot n_e \cdot 41d + CD + AB,$$

wobei die Strecke

$$41d = l_k \cdot [1 - (\sin \alpha)^2 / n_e^2]^{-\frac{1}{2}}$$

die Strecke CD

$$CD = \frac{Z_{CD,1} \cdot Z_{CD,2}}{N_{CD}}$$

mit

$$N_{CD} = n_e \cdot [1 - \sin^2\alpha / n_e^2]^{\frac{1}{2}} + \tfrac{1}{2}[l_k - l_k \cdot \tan(\alpha/2)] \cdot \tan\alpha,$$

$$Z_{CD,1} = -2 \cdot \sin^2\alpha \cdot (l_k - n_e \cdot \sin\alpha^{-1} \cdot (1 - \sin^2\alpha / n_e^2)^{\frac{1}{2}},$$

$$Z_{CD,2} = [l_k/2 + l_k \cdot \tan(\alpha/2)/2 - \cos\alpha^{-1} \cdot [l_k/2 - l_k \cdot \tan(\alpha/2)/2]$$

und die Strecke AB = $\tfrac{1}{2} \cdot [l_k - (l_k \cdot \tan(\alpha/2)] \cdot \tan \alpha$.

Die Parameter **e**, $\mathbf{l_k}$ und $\mathbf{n_e}$ lassen sich nun derart optimieren, daß für einen vorgegebenen Winkelbereich $\alpha$ eine annähernd lineare Weglängenänderung **v** als Wegdifferential erreichbar ist, wie die **Figuren 5** und **6** zeigen. Die Weglängenvariation als zeitliche Ableitung der Weglängenänderung ergibt sich zu

$$v = \frac{ds}{dt} = w \cdot \frac{ds}{d\alpha},$$

wobei **w** die Winkelgeschwindigkeit des rotierenden Elements **15** ist. Die ermittelte zeitliche Weglängenänderung **v** und die mit dem Strahlungsdetektor meßbare Differenzfrequenz ▲f sind über die Relation

$$\blacktriangle f = \frac{2 \cdot f_0 \cdot v}{c}$$

miteinander verknüpft.

Die Weglängenvariation **v** ergibt sich zu

$$v = \frac{Z_{V,1} - Z_{V,2} \cdot Z_{V,3}}{N_{V,1}} - \frac{Z_{V,4} \cdot Z_{V,5}}{N_{V,2}} \, Z_1 - $$

$$2 \cdot \sin\alpha^2 / N_{V,2} \cdot [Z_{V,3} \cdot \cos\alpha + Z_{V,6} \cdot Z_{V,7} \cdot (Z_{V,8} - Z_{V,6})]$$

mit

$$Z_{V,1} = \frac{2 \cdot I_k \cdot \cos\alpha \cdot \sin\alpha}{n_e[I - \sin\alpha^2/n_e^2]^{3/2}} + 2 \cdot \cos\alpha^{-2} \cdot \tfrac{1}{2} \cdot I_k[1 - \tan(\alpha/2)];$$

$$Z_{V,2} = 2 \cdot \cos\alpha \cdot \sin\alpha^3 \cdot [I_k - n_e \cdot \sin\alpha^{-1} \cdot [1 - \sin\alpha^2/n_e^2]^{1/2};$$

$$Z_{V,3} = I_k/2 \cdot [(1 + \tan(\alpha/2) - \cos\alpha^{-1} \cdot (1 - \tan(\alpha/2)];$$

$$N_{V,1} = n_e \cdot (1 - \sin\alpha^2/n_e^2)^{3/2};$$

$$Z_{V,4} = 4 \cdot \cos\alpha \cdot \sin\alpha \cdot (I_k - n_e \cdot \sin\alpha^{-1} \cdot (1 - \sin\alpha^2/n_e^2)^{1/2};$$

$$Z_{V,5} = I_k/2 \cdot [1 + \tan(\alpha/2) - \cos\alpha^{-1} \cdot (1 - \tan(\alpha/2)];$$

$$N_{V,2} = n_e \cdot (1 - \sin\alpha^2/n_e^2);$$

$$Z_1 = \tfrac{1}{2} \cdot [I_k \cdot \sin(\alpha/2)^{-2} \cdot \tan\alpha];$$

$$Z_{V,6} = n_e \cdot \cotg\alpha \cdot \cos\alpha^{-1} \cdot [1 - \sin^2/n_e^2];$$

$$Z_{V,7} = Z_{V,3} - n_e \cdot \cos\alpha^{-1} \cdot [1 - \sin\alpha^2/n_e^2]^{1/2};$$

$$Z_{V,8} = I_k/4 \cdot [\sin(\alpha/2)^{-2} + (\sin(\alpha/2)^{-2} \cdot \sin\alpha^{-1});$$

$$Z_{V,9} = I_k/2 \cdot \sin\alpha^{-1} \cdot \tan\alpha \cdot [1 - \tan(\alpha/2)].$$

Um einen kompakten Aufbau zu erreichen, wird der Abstand des Reflektors **30** von der Rotationsachse **37** des Elements **15** gerade so groß gewählt, daß ein störungsfreies Vorbeirotieren der Kanten **35a - d** gegeben ist.

Wird auf die Beschichtungen **40a** bis **40d** verzichtet, so kann gegenüber der Darstellung in den **Figuren 5** und **6** ein größerer Drehwinkelbereich ▲α des Elements **15** ausgenützt werden. Die für die Interferenz zur Verfügung stehende Intensität ist dann allerdings geringer, aber noch ausreichend, da aufgrund der guten Linearität der Wegdifferenzänderung ▲s/t eine gute Filtermöglichkeit gegeben ist.

Mehrfachreflexionen, welche neben den in den **Figuren 3** und **4** gezeigten Strahlwegen liegen, stören nicht, da sie aufgrund der guten Blendenwirkung der Einkopplung in den Strahlleiter **20b** nicht in diese gelangen.

Zur Signalauswertung wird das mit dem Strahlungsdetektor **7** empfangene Strahlungssignal in ein elektrisches Signal umgewandelt. Dabei wird mit dem Versuchsaufbau die elektrisch Frequenz, welche der Dopplerfrequenzverschiebung ▲f der Strahlung entspricht, herausgefiltert und nur diese verarbeitet, um ein möglichst hohes Signal-Rasch-Verhältnis zu erreichen. Die minimale Bandbreite wird durch die inverse Meßzeit für eine halbe Kohärenzlänge der Strahlungswelle bestimmt. Die Signalauswertung erfolgt in Abhängigkeit der mit einem Winkelgeber vom Element **15** abgenommenen Winkellage des Drehwinkels $\alpha$, welche einer bestimmten Stellung des Elements **15** und damit einer vorgegebenen Wegdifferenz ▲s entspricht.

Falls die verbleibende Nichtlinearität im Ausdruck $ds/d\alpha$ zu größeren Bandbreiten führt, kann entweder mit größeren Filterbandbreiten gearbeitet werden, welche ein reduziertes Signal-Rausch-Verhältnis nach sich ziehen, oder es wird der benutzte Winkelbereich so weit reduziert, bis die minimale Bandbreite wieder erreicht wird. Die letzt genannte Möglichkeit bewirkt eine Verkleinerung des Meßbereichs, welcher aber mit einer Vergrößerung der Würfelkantenlänge $l_k$ kompensiert werden kann.

Befindet sich nun ein für die Strahlung der Strahlungsquelle 3 transparenter Gegenstand **1** im Meßarm **11**, so wird jedesmal dann ein elektrisches Signal mit der Frequenz ▲f der Dopplerfrequenzverschiebung erhalten, wenn die Wege im Meß- und im Referenzarm **11** und **13** gleich lang sind. Die mit dem Element **15** erzeugbare Weglängendifferenz ▲s muß deshalb mindestens so groß sein wie die Dicke **d** des auszumessenden Gegenstands unter Berücksichtigung des Brechungsindexes $n_g$ bzw. $n_e$ für den Gegenstand **1** und für das Element **15**.

In den **Figuren 5** und **6** sind die mit dem obigen Beispiel erreichbaren Wegdifferenzen ▲s sowie in der jeweils darunterliegenden Abbildung die die Weglängenänderung pro Sekunde (Zeiteinheit) ▲s/t bei 2,5 Hz Rotationsfrequenz des Elements 15 über dem Drehwinkel $\alpha$ aufgetragen. Je nach der Breite **a** der Beschichtungen **40a** bis **40d** beginnen die tatsächlichen Werte für die Wegdifferenz ▲s sowie für die Weglängenänderung pro Sekunde ▲s/t bei höheren Winkelwerten (z. B. im Fall von Figur 3 erst bei etwa 20°) und enden bereits bei tieferen als 90° (z. B. etwa 50° im Beispiel). Wird auf die Beschichtungen 40a bis 40d verzichtet, so können größere Winkelbereiche erreicht werden.

Ein Brechungsindex von $n_e$ = 1,5 bzw. 2,5 für das Medium des Elements **15** ist in den **Figuren 5** bzw. **6** Parameter. Aus den **Figuren 5** und **6** ist zu entnehmen, daß für das obige Beispiel mit einem Brechungsindex $n_e$ des Mediums von $n_e$ = 2,5 eine gute Linearität der Weglängenänderung pro Sekunde mit einer Abweichung von lediglich etwa nur 3% erreichbar ist.

Durch die Verwendung der beiden Strahlungsleiter **20a** und **20b** kann nun ein Gegenstand in einem nahezu beliebigen Abstand vom Koppler **17** und der dazugehörenden Auswerteeinheit **10** ausgemessen werden. Wird z. B. die Dicke der menschlichen Kornea bestimmt, so werden bevorzugt der Koppler **17**, die beiden Polarisationskontrolleinheiten **19a** und **19b**, die Strahlungsquelle **3**, der Strahlungsdetektor **7**, der Reflektor **30**, die Linse **31**, das Element **15** und die Auswerteeinheit **10** in einem Gerät untergebracht, in welches zur Weganpassung dann ein vorgegebener Strahlungsleiter **20b** ankoppelbar ist. Die Meßeinheit **23** wird dann unmittelbar vor das menschliche Auge gesetzt und über den Strahlungsleiter **20a** passender Länge mit dem Gerät verbunden. Anstelle der menschlichen Kornea können selbstverständlich auch andere transparente Gegenstände, wie z. B. Folien, Beschichtungen, Platten, Plattenstapel etc. ausgemessen werden.

Mit der erfindungsgemäßen Vorrichtung kann nicht nur die Dicke d einer "Platte", d. h. nicht nur der Abstand von Reflexionen an zwei Oberflächen bestimmt werden, es können auch die Abstände mehrerer übereinanderliegender Oberflächen bestimmt werden. Auch können mit der Vorrichtung Distanzen zu Grenzflächen in einem diffusen Medium oder Ausdehnungen diffuser Zonen bestimmt werden.

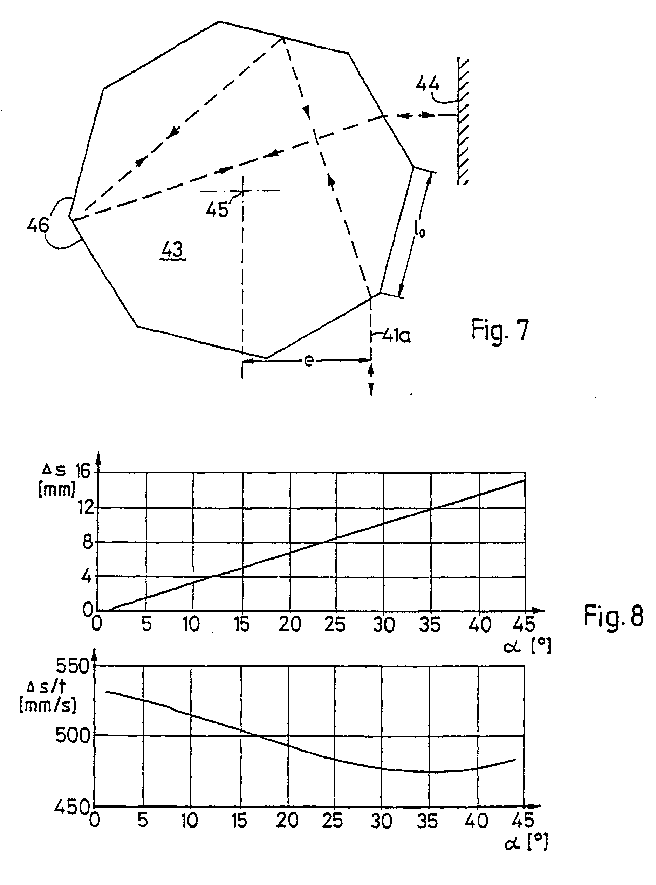

Anstatt des oben beschriebenen viereckigen Würfels als Element **15** können auch andere Zylinder mit einer mehreckigen, ein regelmäßiges Vieleck bildenden Querschnittsfläche, in der der Referenzstrahl zu liegen kommt, verwendet werden. In **Figur 7** z. B. ist ein Oktagon **43** dargestellt. Hierbei ist jedoch ein zum Reflektor **30** analoger Reflektor **44** nicht mehr auf der Seite des in das Element eintretenden Strahls **41a** angeordnet, sondern hierzu um 90° versetzt. **Figur 8** zeigt ein zu **Figur 6** analoges Diagramm für das Oktagon **43** in Figur 7. Die Rotationsfrequenz beträgt hierbei **4** Hz, der Brechungsindex $n_e$ = 2,5 (ZnSe) und der Abstand **e** des eintretenden Strahls **41a** von der Rotationsachse **45** beträgt **e** = 12 mm und die Flächenbreite $l_0$ = 30 mm. Bevorzugt werden hier die Zylindermantelflächen **46** mit einer Beschichtung versehen, deren Reflexionsgrad stark vom Einfallswinkel des Strahls abhängt.

**Patentansprüche**

1. Vorrichtung zur Messung der Dicke transparenter Gegenstände **(1)**, mit einer Strahlungsquelle **(3)** kurzer Kohärenzlänge und einem Michelson-Interferometer **(5)**, in dessen Meßarm **(11)** der auszumessende Gegenstand **(1)** einbringbar ist, sowie einem die optische Weglänge infolge Eigenrotation um eine Achse **(37)** periodisch verändernden Weglängenvariationselement **(15)** im Meß- und/oder Referenzarm **(11, 13), dadurch gekennzeichnet, daß** die Querschnittsfläche **(34)** des den vom Interferometer **(5)** ausgehenden Strahl **(41b - 41d)** aufnehmenden Elements **(15)** wenigstens viereckig ist, der Strahl **(41b - 41d)** im Element **(15)** wenigstens zwei Reflexionen an den Elementinnenflächen hat und wenigstens eine Teilintensität des aus dem Element **(15)** austretenden Strahls **(41e)** durch dieses Element **(15)** hindurch, rückreflektierbar ist, bevorzugt in sich selbst.

**2.** Vorrichtung nach Anspruch 1, **dadurch gekennzeichnet, daß** die den Strahl **(41b - 41d)** aufnehmende Querschnittsfläche **(34)** des Elements **(15)** ein regelmäßiges Vieleck ist.

**3.** Vorrichtung nach Anspruch 1 oder 2, **dadurch gekennzeichnet, daß** die Rotationsachse **(37)** des Elements **(15)** senkrecht auf der den Strahl **(41b - 41d)** aufnehmenden Querschnittsfläche **(34)** steht und der Strahleintrittsort des vom Koppler **(17)** des Interferometers **(5)** kommenden Strahls **(41a)** in das Element **(15)** zur Rotationsachse **(37)** um einen vorgegebenen Abstand **(e)** seitlich versetzt angeordnet ist.

**4.** Vorrichtung nach Anspruch 3, **dadurch gekennzeichnet, daß** der Reflektor **(30)** des Referenzarms **(13)** derart seitlich versetzt ist, daß der vom Koppler **(17)** kommende Strahl **(41a)** oder der auszumessende Gegenstand **(1)** gerade noch das Element **(15)** trifft.

**5.** Vorrichtung nach einem der Ansprüche 1 bis 4, **dadurch gekennzeichnet, daß** die Mantelflächen **(39a - 39d)** des Elements **(15)** wenigstens teilweise vergütet sind, wobei bevorzugt jeweils nur ein Mantelflächenbereich **(40a - 40d)**, beginnend an jeder Mantelkante **(35a - 35d)**, für nur denjenigen innerhalb des Elements **(15)** reflektierbaren Strahl **(41c, 41d)**, der mit dem dazugehörenden einfallenden Strahl **(41b, 41c)** einen spitzen Winkel bildet, reflektierend beschichtet ist, um eine möglichst verlustvermindernde Strahlführung zu erreichen.

**6.** Vorrichtung nach einem der Ansprüche 1 bis 5, bevorzugt nach Anspruch 2 und 3, **dadurch gekennzeichnet, daß** der optische Brechungsindex **(n)** des Elements **(15)**, dessen Abmessungen **(l)** und bevorzugt der seitliche Versatz **(e)** des in das Element (15) einzustrahlenden Strahls **(41a)** derart ausgewählt werden, daß die Weglängenänderung in Abhängigkeit des Rotationswinkels ($\alpha$) des Elements **(15)** möglichst konstant ist.

**7.** Vorrichtung nach einem der Ansprüche 1 bis 6, **dadurch gekennzeichnet, daß** der Referenz- und/oder der Meßarm **(13, 11)** zwischen dem Koppler **(17)** und einer Meßund/oder Referenzeinheit **(23, 29)** einen, bevorzugt je einen Strahlungsleiter **(20a, 20b)** mit einer Polarisationskontrolleinheit **(19a, 19b)** hat bzw. haben, um insbesondere die Meßstrahlung auf einfache Art und Weise auch an auszumessende Gegenstände **(1)** mit unterschiedlicher geometrischer Lage zum Koppler **(17)** bzw. zu den restlichen Teilen der Vorrichtung heranzubringen.

**8.** Vorrichtung nach Anspruch 7, **dadurch gekennzeichnet, daß** optische Wegstrecken in Luft, Geometrie und Material der Strahlungsleiter **(20a, 20b)**, sowie sämtlicher optischen Elemente **(19a, 19b, 25, 26, 31, 15)** im Referenz- und Meßarm **(13, 11)** derart ausgewählt sind, daß ein Unterschied in der Dispersion in beiden Armen **(13, 11)** vernachlässigbar ist und in bevorzugter Weise die Strahlungsleiter **(20a, 20b)** auswechselbar angeordnet sind.

**9.** Vorrichtung nach Anspruch 7 oder 8, **gekennzeichnet durch** je einen Kollimator **(25, 31),** mit dem die aus den Strahlungsleitern **(19a, 19b)** in die Referenz- und Meßeinheit **(29, 23)** des Referenz- bzw.Meßarms **(13, 11)** austretende Strahlung **(41a)** kollimierbar ist, eine Fokussierlinse **(26),** mit der die kollimierte Strahlung in der Meßeinheit auf den Gegenstand (l) fokussierbar ist,und insbesondere eine frequenzselektive Signalauswerteeinheit **(10),** mit der lediglich die mit dem Weglängenvariationselement **(15) durch** Überlagerung der Referenz- und Meßstrahlung im Koppler **(17)** erzeugte Dopplerfrequenz in Abhängigkeit des Drehwinkels ($\alpha$) des Elements **(15)** zur Erhöhung des Signal-Rausch-Abstandes verarbeitbar ist.

**10.** Verwendung der Vorrichtung nach einem der Ansprüche 1 bis 9, **dadurch gekennzeichnet, daß** der Gegenstand **(1)** die Kornea eines insbesondere lebenden Auges ist.

### Claims

**1.** Apparatus for measuring the thickness of transparent objects (1), having a radiation source (3) with a short coherence length and a Michelson interferometer (5) into whose measuring arm (11) the object (1) to be measured can be introduced, as well as a path length variation element (15) in the measuring and/or reference arm (11, 13), which path length variation element (15) periodically changes the optical path length owing to its own rotation about an axis (37), **characterised in that** the cross-sectional surface (34) of the element (15) receiving the beam (41b - 41d) coming out of the interferometer (5) is at least four-sided, the beam (41b - 41d) in the element (15) is reflected at least twice on the inner faces of the element and at least a partial intensity of the beam (41e) exiting from the element (15) can be reflected back, preferably into itself, through the element (15).

**2.** Apparatus according to claim 1, **characterised in that** the cross-sectional surface (34) of the element (15), which surface (34) receives the beam (41b - 41d), is a regular polygon.

3. Apparatus according to claim 1 or 2, **characterised in that** the rotation axis (37) of the element (15) stands perpendicularly on the cross-sectional surface (34) that receives the beam (41b - 41d) and the site at which the beam (41a) coming from the coupler (17) of the interferometer (5) enters the element (15) is arranged displaced in sideways direction with respect to the rotation axis (37) by a preset distance (e).

4. Apparatus according to claim 3, **characterised in that** the reflector (30) of the reference arm (13) is displaced sideways in such a manner that the beam (41a) coming from the coupler (17) or the object (1) to be measured just meets the element (15).

5. Apparatus according to any one of claims 1 to 4, **characterised in that** the exterior surfaces (39a - 39d) of the element (15) are at least partially coated, preferably only one exterior surface region (40a - 40d) in each case, starting at each exterior edge (35a - 35d), being reflectively coated only for the beam (41c, 41d), reflectable within the element (15), that forms an acute angle with the corresponding incident beam (41b, 41c), in order to achieve beam guidance that is as loss-reducing as possible.

6. Apparatus according to any one of claims 1 to 5, preferably according to claims 2 and 3, **characterised in that** the optical refractive index (n) of the element (15), its dimensions (1), and preferably the sideways displacement (e) of the beam (41a) that is to be irradiated into the element (15), are selected in such a manner that the path length modification is as constant as possible in dependence upon the rotation angle ($\alpha$) of the element (15).

7. Apparatus according to any one of claims 1 to 6, **characterised in that** the reference arm and/or the measuring arm (13, 11) between the coupler (17) and a measuring and/or reference unit (23, 29) has one or preferably each has a radiation guide (20a, 20b) with a polarisation control unit (19a, 19b) to bring, particularly, the measuring radiation in a simple manner also to objects (1) to be measured with different geometric positions with respect to the coupler (17) or with respect to the remaining parts of the apparatus.

8. Apparatus according to claim 7, **characterised in that** the optical path distances in air, the geometry, and the material of the radiation guides (20a, 20b) as well as of all the optical elements (19a, 19b, 25, 26, 31, 15) in the reference and measuring arm (13, 11) are selected in such a manner that a difference in the dispersion in the two arms (13, 11) is negligible and the radiation guides (20a, 20b) are preferably arranged in such a manner that they can be exchanged.

9. Apparatus according to claim 7 or 8, **characterised by** a respective collimator (25, 31) that allows the collimation of the radiation (41a) coming out of the radiation guides (19a, 19b) into the reference and measuring unit (29, 23) of the reference and measuring arm (13, 11), a focusing lens (26) that allows the focusing of the collimated radiation in the measuring unit onto the object (1), and particularly a frequency-selective signal evaluation unit (10) that allows only the Doppler frequency generated with the path length variation element (15) by superposing the reference and measuring radiation in the coupler (17) to be processed in dependence upon the rotation angle ($\alpha$) of the element (15) in order to increase the signal-noise distance.

10. Use of the apparatus according to any one of claims 1 to 9, **characterised in that** the object (1) is the cornea of, in particular, a living eye.

**Revendications**

1. Dispositif pour mesurer l'épaisseur d'objets transparents (1), comportant une source de rayonnement (3) ayant une courte longueur de cohérence et un interféromètre de Michelson (5), dans le bras de mesure (11) duquel peut être introduit l'objet à mesurer (1), ainsi qu'un élément (15) de modification de la longueur d'onde, qui modifie périodiquement la longueur d'onde optique sur la base d'une rotation propre autour d'un axe (37), dans le bras de mesure et/ou le bras de référence (11, 13), **caractérisé en ce que** la surface en coupe transversale (34) de l'élément (15), qui reçoit le faisceau (41b-41d) émis par l'interféromètre (5), est pourvu d'au moins quatre côtés, que le faisceau (41b-41d) subit dans l'élément (15) au moins deux réflexions sur les surfaces internes de l'élément et qu'au moins une intensité partielle du faisceau (41e) qui sort de l'élément (15), subit une rétroréflexion sur lui-même, à travers cet élément (15).

**2.** Dispositif selon la revendication 1, **caractérisé en ce que** la surface en coupe transversale (34), qui reçoit le faisceau (41b-41d), de l'élément (15) est un quadrilatère régulier.

**3.** Dispositif selon la revendication 1 ou 2, **caractérisé en ce que** l'axe de rotation (37) de l'élément (15) est perpendiculaire à la surface en coupe transversale (34) qui reçoit le faisceau (41b-41d) et que le poids d'entrée du faisceau (41a), qui arrive du coupleur (17) de l'interféromètre (5), dans l'élément (15) est disposé d'une manière décalée latéralement d'une distance prédéterminée (e) par rapport à l'axe de rotation (37).

**4.** Dispositif selon la revendication 3, **caractérisé en ce que** le réflecteur (30) du bras de référence (13) est décalé latéralement de telle sorte que le faisceau (41a) qui arrive du coupleur (17), ou l'objet à mesurer (1) atteint encore précisément l'élément (15).

**5.** Dispositif selon l'une des revendications 1 à 4, **caractérisé en ce que** les surfaces enveloppes (39a-39d) de l'élément, (15) sont au moins partiellement métallisées, auquel cas de préférence respectivement seule une partie (40a-40d) de la surface enveloppe, qui commence au niveau de chaque bord d'enveloppe (35a-35d), est recouvert d'un revêtement réfléchissant uniquement pour le faisceau (41c, 41d), qui peut être réfléchi à l'intérieur de l'élément (15) et fait un angle aigu avec le faisceau incident associé (41b, 41c), pour obtenir un guidage du rayonnement, qui réduit autant que cela est possible les pertes.

**6.** Dispositif selon l'une des revendications 1 à 5, de préférence selon les revendications 2 et 3, **caractérisé en ce que** l'indice de réfraction optique (n) de l'élément (15), ses dimensions (1) et de préférence le décalage latéral (e) du faisceau (41a) devant être introduit dans l'élément (15), sont choisis de telle sorte que la variation de la longueur d'onde en fonction de l'angle de rotation ($\alpha$) de l'élément (15) est aussi constante que possible.

**7.** Dispositif selon l'une des revendications 1 à 6, **caractérisé en ce que** le bras de référence ou le bras de mesure (13, 11) entre le coupleur (17) et une unité de mesure et/ou de référence (23, 29) possèdent un et de préférence respectivement chacun un guide de rayonnement (20a, 20b) comportant une unité de contrôle de polarisation (19a, 19b), de manière à amener notamment le rayonnement de mesure d'une manière simple également aux objets à mesurer (1) avec une position géométrique différente par rapport au coupleur (17) ou aux autres parties du dispositif.

**8.** Dispositif selon la revendication 7, **caractérisé en ce que** des sections de trajets optiques dans l'air, la géométrie et le matériau des guides de rayonnement (20a, 20b) ainsi que de tous les éléments optiques (19a, 19b, 25, 26, 31, 15) dans le bras de référence et dans le bras de mesure (13, 11) sont choisis de telle sorte qu'une différence des dispersions dans les deux bras (13, 11) est négligeable et que de préférence les guides de rayonnement (20a, 20b) sont disposés de façon interchangeable.

**9.** Dispositif selon la revendication 7 ou 8, **caractérisé** respectivement par un collimateur (25, 31), au moyen duquel le rayonnement (41a), qui sort des guides de rayonnement (19a, 19b) pour pénétrer dans l'unité de référence et dans l'unité de mesure (29, 23) du bras de référence et du bras de mesure (13, 11), peut être collimaté, une lentille de focalisation (26), au moyen de laquelle le rayonnement collimaté peut être focalisé dans l'unité de mesure sur l'objet (1), et notamment une unité (10) d'évaluation du signal, sélective en fréquence et au moyen de laquelle la fréquence Doppler, qui est produite avec l'élément (15) de modification de la longueur d'onde par superposition du rayonnement de référence et du rayonnement de mesure dans le coupleur (17), peut être traitée en fonction de l'angle de rotation ($\alpha$) de l'élément (15) de manière à accroître le rapport signal/bruit.

**10.** Utilisation du dispositif selon l'une des revendications 1 à 9, **caractérisée en ce que** l'objet (1) est la cornée d'un oeil, notamment vivant.

Fig. 2

Fig. 3

Fig. 4

Fig. 1

Fig. 5

Fig. 6

Fig. 7

Fig. 8